**Europäisches Patentamt**

(19) **European Patent Office**

**Office européen des brevets**

(11) Numéro de publication: **0 073 747**
**A2**

# (12) DEMANDE DE BREVET EUROPEEN

(21) Numéro de dépôt: 82870046.8

(22) Date de dépôt: 26.08.82

(51) Int. Cl.³: **C 12 P 1/00,** C 12 M 1/02
// C12P5/02, C12P7/06

---

(30) Priorité: 26.08.81 BE 205764

(71) Demandeur: **ATELIERS DE CONSTRUCTIONS ELECTRIQUES DE CHARLEROI (ACEC) Société Anonyme, 54, Chaussée de Charleroi, B-1060 Bruxelles (BE)**

(43) Date de publication de la demande: 09.03.83
**Bulletin 83/10**

(72) Inventeur: **de Baere, Luc, Martelaerslaan, 98, B-9000 Gand (BE)**

(84) Etats contractants désignés: **AT BE CH DE FR GB IT LI LU NL SE**

(74) Mandataire: **Bossard, Franz et al, ACEC - Service des Brevets Boîte Postale 4, B-6000 Charleroi (BE)**

---

(54) **Procédé et installation de production de composés liquides ou gazeux dans un réacteur microbiel.**

(57) Procédé de production de composé liquides ou gazeux dans un réacteur microbiel dans lequel un liquide chargé de solides est amené dans un dispositif (2) séparant une fraction limpide d'une fraction épaisse du liquide, la fraction épaisse du liquide étant chauffée à une température élevée assurant la solubilisation d'au moins une partie des composants solides de cette fraction et ensuite remélangée avec la fraction limpide et amenée dans le réacteur (7).

PROCEDE ET INSTALLATION DE PRODUCTION DE COMPOSES LIQUIDES OU GAZEUX
DANS UN REACTEUR MICROBIEL.

La présente invention a pour objet un procédé et une installation de production de composés liquides ou gazeux dans un réacteur microbiel notamment mésophile, ou thermophile, fonctionnant à des températures aux environs de respectivement 35°C ou 55°C.

L'invention est applicable non seulement à la production de composés gazeux, par exemple à la méthanisation de boues, mais aussi à d'autres procédés de production microbielle qui fonctionnement à des températures entre 30 et 75°C, telle la production de composés liquides, par exemple d'éthanol , à partir d'influents chargés de solides .

Il est déjà connu de traiter des liquides chargés de solides dans un réacteur méthaniseur dans lequel on maintient une température d'environ 40°C et dans lequel les liquides usés séjournent en moyenne un ou deux jours. Un temps de séjour moyen d'un jour est suffisant si les liquides ne véhiculent pas de boues. Par contre, un séjour d'une ou plusieurs semaines dans le réacteur est nécessaire si les liquides véhiculent une proportion très importante de boues.

L'invention a pour but d'utiliser d'une manière particulièrement efficace la chaleur nécessaire pour porter un influent chargé de matières solides à une température optimum en vue d'une production de composants liquides ou gazeux maximum.

Un autre but est d'augmenter l'activité méthanogène dans un processus de méthanisation d'un influent chargé de boues très peu solubles, c'est-à-dire de réduire le temps de séjour des liquides dans le réacteur afin de réduire l'encombrement et le coût d'une installation.

Le procédé pour la décomposition de boues dans un réacteur suivant l'invention est caractérisé en ce qu'un liquide chargé de solides est amené dans un dispositif séparant une fraction limpide d'une fraction épaisse des liquides, en ce que la fraction épaisse est chauffée à une température élevée assurant la solubilisation d'au moins une partie des composants solides de cette fraction et en ce que la fraction ainsi traitée est remélangée avec la fraction limpide et amenée dans le réacteur. De préférence, la proportion de la fraction épaisse et la température à laquelle est portée cette fraction épaisse pour le traitement

39.22/1852

thermique sont choisies de telle manière qu'après mélange de cette fractions avec la fraction liquide froide, la température du mélange est la température optimum pour un débit maximum de composés liquides ou gazeux formés dans le réacteur.

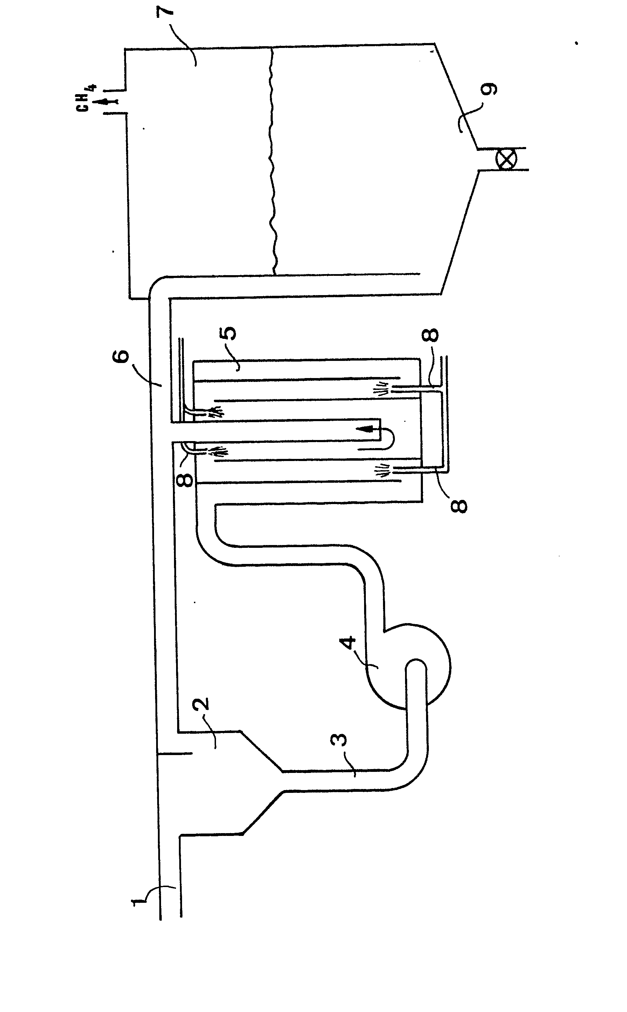

L'invention est décrite ci-dessous par rapport à un exemple d'une installation dont le schéma figure au dessin annexé.

Du lisier est amené par une conduite 1 dans u n dispositif de séparation qui peut être un filtre, une centrifuge ou notamment un bassin décanteur 2. Dans ce bassin, les particules solides, lourdes s'accumulent dans le fond et constituent une fraction épaisse. Cette dernière est évacuée par une conduite 3 à l'aide d'une pompe 4. La pompe 4 peut être équipée d'un dispositif de désintégration pour les boues, par exemple un grillage ou des couteaux disposés sur la périphérie d'entrée de la pompe. De la pompe 4, la fraction épaisse est envoyée dans un dispositif de traitement thermique en continu 5 ou un autoclave à fonctionnement discontinu dans lequel elle est chauffée à une température élevée, par exemple entre 80 et 250°C afin de rendre soluble une proportion importante de cette boue. La proportion de boue envoyée à travers le dispositif de traitement thermique par rapport à la proportion de la fraction liquide est réglée par la vitesse de rotation de la pompe 4. Le choix de la température dépend de la composition des boues. S'il s'agit de boues facilement solubilisables une température entre 80 et 120°C peut déjà suffire. Par contre, une température entre 130 et 150°C convient pour une boue composée de particules monocellulaires et une température de 180 à 200°C permet de solubiliser des substances à parois cellulosiques. Une température de 200 à 220° C en milieu oxydant permet de solubiliser preque toutes les boues en matière organique, mais au delà de ces températures, des effets de polymerisation sont à craindre de sorte qu'au delà de 250°C l'effet de solubilisation est en général anéanti par des effets de polymérisation.

La fraction quittant le dispositif de traitement thermique 5, déterminée par la vitesse de la pompe 4 est injectée dans une conduite 6 véhiculant la fraction limpide non chauffée provenant du décanteur 2. Si une proportion de fraction limpide d'un quart est soumis à un traitement de 140°C dans le dispositif 5, cette proportion est mélangée avec trois quarts de la fraction limpide à 10°C
en aval du dispositif 5, ce qui amène la température du mélange à environ 41°C qui pour certaines couches de bactéries anaérobies constitue
une température optimum pour assurer un débit de méthane maximum dans le
réacteur 7 recevant ce mélange. Le dispositif de traitement thermique
5 est de préférence agencé de telle manière que toute fuite de chaleur
est récupérée pour le chauffage de la fraction qui traverse le dispositif 5.

La pompe 4 peut être entraînée par exemple par un moteur électrique
ou par un moteur à explosion alimenté par le méthane produit dans le
réacteur méthaniseur. Le chauffage dans le dispositif de traitement
thermique 5 peut être réalisé soit par la combustion du méthane produit
chauffant directement le dispositif 5 ou engendrant d'abord de la vapeur
dans une chaudière non représentée. Cette vapeur est ensuite injectée à
travers des tuyères 8 dans le dispositif de traitement thermique 5. Si
le moteur de la pompe 4 est un moteur à explosion, il est possible
aussi d'injecter directement les gaz d'échappement de ce moteur à travers les tuyères 8. La vapeur d'eau ou les gaz injectés à travers les
tuyères 8 dans le dispositif de traitement thermique 5 permettent d'y
atteindre une température entre 80°C et 250°C.

Le réacteur méthanigène 7 peut être un réacteur infiniment mélangé
suivi d'un décateur ou comme cela est montré sur le dessin un réacteur
à flux ascendant. L'effluent du réacteur infiniment mélangé ou l'effluent boueux récolté dans le fond du décanteur 9 du réacteur 7 peuvent
être utilisés, au moins en partie comme boue d'inoculation méthanigène
dans d'autres procédés de biodégradation de boues ou de déchets organiques putrescibles solides et permettre ainsi une accélération notable
de ceux-ci.

En lieu et place de lisier, l'influent envoyé dans le dispositif
de séparation 2, peut être aussi des rejets de criées, de fruits ou
légumes, ou d'autres résidus agro-alimentaires ou encore des boues
activées provenant d'un système d'épuration d'eaux d'égouts dont les
substances non pompables ont été éliminées au préalable.

**0073747**

# REVENDICATIONS

1. Procédé de production de composés liquides ou gazeux dans un réacteur microbiel,

caractérisé en ce qu'un liquide chargé de solides est amené dans un dispositif (2) séparant une fraction limpide d'une fraction épaisse du liquide, en ce que la fraction épaisse du liquide est chauffée à une température élevée assurant la solubilisation d'au moins une partie des composants solides de cette fraction et en ce que la fraction ainsi traitée est remélangée avec la fraction limpide et amenée dans le réacteur (7).

2. Procédé suivant la revendication 1, caractérisé en ce que la proportion de la fraction épaisse et la température maximum à laquelle elle est soumise sont choisie de telle manière que la température du mélange entre fraction limpide et fraction épaisse après chauffage est égale à une température voisine de la température optimum pour un débit maximum de composés liquides ou gazeux produit dans le réacteur.

3. Procédé suivant une des revendications précédentes, caractérisé en ce qu'on utilise l'effluent du réacteur (7) comme boue d'inoculation méthanigène dans d'autres procédés de biodégradation de boues ou de déchets organiques putrescibles solides.

4. Installation pour l'exécution d'un procédé suivant une des revendications 1 à 3, caractérisée en ce qu'elle comprend un dispositif séparateur (2) disposé dans la conduite d'entrée (1), une pompe 4 extrayant une fraction épaisse du séparateur (2) et la refoulant dans un dispositif de traitement thermique (5) chauffé, un espace de mélange (6) alimenté par la sortie du dispositif de traitement thermique (5) d'une part et la sortie pour la fraction limpide du séparateur (2), d'autre part le dit espace de mélange formant au moins en partie conduite d'entrée du réacteur (7).

5. Installation suivant la revendication 4, caractérisée en ce que la pompe et le dispositif de traitement thermique sont remplacés par une vis d'extrusion à pas décroissant vers l'aval dans un conduit chauffé.

39.22/1852.

CH$_4$

1
2
3
4
5
6
7
8
8
8
9

0073747